# FASCICULE DE BREVET EUROPEEN

(11) **EP 2 734 495 B1**
(45) Date de publication et mention de la délivrance du brevet: **23.08.2017**
(21) Numéro de dépôt: 12744073.3
(22) Date de dépôt: 10.07.2012
(51) Int. Cl.: C07C 51/285, C07C 51/36, C07C 51/34, C07C 51/377, C07C 59/01, C07C 57/12, C07C 55/02, C07C 227/18, C07C 229/08, C07C 253/22, C07C 255/23, C07C 253/30, C07C 255/19, C07C 227/16, C07C 227/28

(54) **PROCEDE DE SYNTHESE D'ACIDES OMEGA-FONCTIONNALISES A PARTIR D'ACIDES OU D'ESTERS GRAS**
VERFAHREN ZUR SYNTHESE VON OMEGA-FUNKTIONALISIERTEN SÄUREN AUS FETTSÄUREN ODER FETTSÄUREESTERN
PROCESS FOR SYNTHESIZING OMEGA-FUNCTIONALIZED ACIDS FROM FATTY ACIDS OR FATTY ESTERS

(30) Priorité: 19.07.2011 FR 1156526
(43) Date de publication de la demande: 28.05.2014
(73) Titulaire: Arkema France, 92700 Colombes (FR)
(72) Inventeur: DUBOIS, Jean-Luc, 69390 Millery (FR)
(74) Mandataire: Killis, Andréas
(86) Numéro de dépôt international: PCT/FR2012/051627
(87) Numéro de publication internationale: WO 2013/011226

(56) Documents cités:
- GB-A- 741 739
- US-A- 2 813 113
- PERKINS R B ET AL: "Nylon-9 from Unsaturated Fatty Derivatives: Preparation and Characterization", JOURNAL OF THE AMERICAN OIL CHEMISTS' SOCIETY, SPRINGER, BERLIN, DE, vol. 52, 1 novembre 1975 (1975-11-01), pages 473-477, XP002545488, ISSN: 0003-021X, DOI: 10.1007/BF02637493
- JOSÉ M. CONCELLON ET AL: "General, Stereoselective Synthesis of ( Z )-[beta],[gamma]-Unsaturated Nitriles Promoted by Samarium Diiodide", ORGANIC LETTERS, vol. 10, no. 20, 16 October 2008 (2008-10-16), pages 4549-4552, XP055301085, US ISSN: 1523-7060, DOI: 10.1021/ol801752m
- WERNER G KNIPPRATH ET AL: "Synthesis of-3-hexadecenoic acid and of-3-hexadecenoic-1-Cacid", LIPIDS, SPRINGER-VERLAG, BERLIN/HEIDELBERG, vol. 1, no. 1, 1 January 1966 (1966-01-01) , pages 81-84, XP035175622, ISSN: 1558-9307, DOI: 10.1007/BF02668128

## Description

Le travail qui a conduit à cette invention a reçu un financement de la part de l'Union Européenne dans le cadre du 7ème Programme Cadre (FP7/2007-2013) sous le numéro de projet N°241718 EUROBIOREF.

La présente invention a pour objet un procédé de synthèse d'acides ω-fonctionnalisés de formule R-(CH₂)ₙ-COOH dans laquelle R représente COOH ou NH₂CH₂ à partir d'une charge d'origine naturelle contenant des acides gras hydroxylés.

L'industrie des polyamides utilise toute une gamme de monomères tels que les diacides, les diamines ainsi que les ω-amino-acides à longue chaîne. Ces polyamides, usuellement appelés Nylon sont caractérisés par la longueur de chaîne méthylène (-CH₂)ₙ séparant deux fonctions amide -CO-NH-. C'est ainsi que sont connus les Nylon-6, Nylon 6-6, Nylon 6-10, Nylon 7, Nylon 8, Nylon 9, Nylon 11, Nylon 13, etc ...

Ces monomères sont par exemple fabriqués par voie de synthèse chimique en utilisant notamment comme matière première des oléfines en C₂ à C₄, des cyclo-alcanes ou du benzène, mais aussi des acides gras comportant le plus souvent 18 atomes de carbone, issus d'huiles naturelles insaturées telles que notamment les huiles de soja, de maïs, de lin, de palme, etc ...

L'évolution actuelle en matière d'environnement conduit dans les domaines de l'énergie et de la chimie à privilégier l'exploitation des matières premières naturelles provenant d'une source renouvelable. C'est la raison pour laquelle certains travaux ont été repris pour élaborer sur le plan industriel des procédés utilisant des acides/esters gras comme matière première de fabrication de ces monomères.

Les principaux travaux ont porté sur la synthèse de l'acide amino-9-nonanoïque qui est le précurseur du Nylon 9 à partir de l'acide oléique d'origine naturelle. En ce qui concerne ce monomère particulier, on peut citer l'ouvrage « n-Nylons, Their Synthesis, Structure and Properties » - 1997, Ed.J. Wiley et Sons dont le chapitre 2.9 (pages 381 à 389) est consacré au 9-Nylon.

En ce qui concerne la synthèse des monomères supérieurs tels que par exemple ceux conduisant d'une part au Nylon 11 ou au Nylon 13 et d'autre part aux diacides supérieurs, on ne trouve que peu de littérature et a fortiori de réalisations industrielles. Elles sont tournées presque exclusivement vers la transformation d'huiles naturelles : huile de ricin, d'olive, de soja, de tournesol, de palme, etc ... contenant des acides gras insaturés en C₁₈ tels que les acides oléique et ricinoléique, l'huile de lesquerella contenant l'acide lesquérolique en C₂₀, l'huile de colza érucique, l'huile de lunaire ou l'huile de crambe contenant de l'acide érucique en C₂₂.

Un des rares exemples de procédé industriel utilisant un acide gras comme matière première est celui de la fabrication à partir de l'acide ricinoléique, extrait de l'huile de ricin, de l'acide amino-11-undécanoïque, qui est à la base de la synthèse du Rilsan 11^{®}. Ce procédé est décrit dans l'ouvrage «Les Procédés de Pétrochimie » de A. Chauvel et al., paru aux Editions TECHNIP (1986). L'acide amino-11-undécanoïque est obtenu en plusieurs étapes. La première consiste en une méthanolyse de l'huile de ricin en milieu basique produisant le ricinoléate de méthyle qui est ensuite soumis à une pyrolyse pour obtenir, d'une part l'heptanaldéhyde et, d'autre part, l'undécylénate de méthyle. Ce dernier est passé en forme acide par hydrolyse. Ensuite, l'acide formé est soumis à une hydrobromuration pour donner l'acide ω-bromé d'où l'on passe par amination à l'acide amino-11-undécanoïque.

L'utilisation des acides gras hydroxylés comme matière première de synthèse d'amino-acides est peu courante en dehors de l'exemple industriel mentionné ci-dessus. On peut cependant citer la demande de brevet FR 2912741 au nom de la demanderesse qui décrit la synthèse des acides 11-aminoundécanoïque et 12-aminododécanoïque par un procédé utilisant notamment la métathèse.

Les diacides (ou diesters) saturés les plus connus sont ceux comportant des chaînes comportant de 4 à 6 atomes de carbone tels que les acides succinique (C₄), glutarique (C₅), adipique (C₆). Ils sont généralement synthétisés par voie chimique ou plus récemment par voie de fermentation pour l'acide succinique.

En revanche, les diacides à chaîne longue sont obtenus soit par fermentation de paraffines ou d'acides gras ou encore par oxydation de cycloalcane comme dans le cas de l'oxydation du cyclododécane en acide dodécanedioïque ou par craquage en milieu basique de l'acide ricinoléique pour donner l'acide sébacique (diacide en C₁₀) ou de l'acide lesquérolique pour donner le diacide en C₁₂, acide dodécanedioïque.

Les propriétés, synthèses et utilisations de ces diacides sont décrites dans l'Encyclopédie ULLMANN, Vol. A8, pages 526-536. Les diacides saturés ou insaturés sont généralement obtenus industriellement selon les différentes méthodes mentionnées dans cette référence. A côté des synthèses par condensation ou fermentation, on retrouve les méthodes par dégradation telles que notamment l'ozonolyse, l'oxydation ou le craquage des acides gras insaturés éventuellement hydroxylés (pages 526, 527 et 532 de cette publication). Dans les demandes de brevet publiées sous les numéros FR 2917406 et FR 2921363, la demanderesse décrit ce type de synthèse appliquée aux acides gras insaturés.

L'utilisation des huiles naturelles comme source d'acides gras insaturés pose un délicat problème dans la mesure où elles sont constituées d'un mélange de divers acides saturés et insaturés, éventuellement poly-insaturés, qui entraîne la formation au cours du traitement, notamment lorsqu'on met en oeuvre la coupure oxydante, de multiples sous-produits dus à la présence d'insaturations multiples qui nécessitent des traitements de séparation/purification coûteux. Ceci est vrai tout aussi bien pour les acides gras que pour les acides gras hydroxylés.

Le problème posé par la présence au sein de l'huile de divers acides gras insaturés se présente avec d'autant plus d'acuité que l'on est amené dans le futur, pour améliorer les productivités, à utiliser de plus en plus des plantes OGM.

Pour illustrer ce phénomène, on peut citer la publication de Linnaeus Plant Science (Jack Grushcow, « Optimising Oil Seed Potential for Industrial Application », Renewable resources and biorefineries conference 6-8 September 2006, The University of York, YORK UK, et Jack Grushcow, « Industrial Oil Seed Opportunities », Soy20-20, September 2004, Annual Meeting) qui décrit le mécanisme de formation des divers acides gras hydroxylés synthétisés dans un processus utilisant comme plante modèle *Arabidopsis thaliana.* Selon ce processus, l'acide oléique, principal acide produit, peut être transformé par hydroxylation en acide ricinoléique lui-même transformé par déshydrogénation en acide densipolique, ces deux derniers étant susceptibles par « élongation » de former respectivement les acides lesquérolique et auricolique.

US 2 813 113 décrit un procédé de préparation d'acide azélaïque par ozonolyse de l'acide oléique.

GB 741 139 décrit un procédé de préparation d'acide 9-amino nonanoïque par ammoniation de l'acide oléique en oléonitrile, suivi d'ozonolyse avec formation de l'acide 9-cyanooctanoïque qui est hydrogéné pour conduire à l'acide 9-aminononanoïque.

Le Journal of the American Oil Chemists' Society, Vol. 52, 1 novembre 1975, pp. 473-477 décrit un procédé pour l'acide 9-aminononanoïque par méthanolyse de l'huile de soja et ensuite ozonolyse réductrice pour obtenir l'acide 9-aldéhyde azélaïque qui est nitrilé en acide 9-cyanooctanoïque et ensuite hydrogéné pour conduire au produit visé.

L'invention vise à pallier ces inconvénients en utilisant comme matière première une huile naturelle riche en acides gras insaturés hydroxylés qui sont transformés en acides gras saturés hydroxylés, ces derniers étant alors déshydratés en acides gras mono-insaturés éventuellement convertis en nitriles et soumis ensuite à une coupure oxydante conduisant aux acides ω-fonctionnalisés, susceptibles d'être transformés en dinitriles.

Pour simplifier l'exposé, l'expression « acides gras hydroxylés insaturés » désignera les acides qu'ils soient sous forme acide, sous forme ester ou sous forme ester de polyol correspondant en pratique dans ce dernier cas à l'huile brute.

La présente invention a pour objet un procédé de synthèse d'acides ω-fonctionnalisés de formule R-(CH₂)ₙ-COOH dans laquelle R représente COOH ou NH₂CH₂ et n un entier compris entre 9 et 12 à partir d'une charge d'origine naturelle contenant des acides gras hydroxylés insaturés sous forme acide, ester (comme méthylique) ou ester de polyol (comme huile glycéride mais pour des raisons de facilité de lecture, par la suite on parlera d'acide pour désigner aussi bien les acides et les esters d'alcools ou de polyols) comportant au moins 18 atomes de carbone par molécule, comportant les étapes suivantes :
a) hydrogénation des acides gras hydroxylés insaturés conduisant à des acides gras hydroxylés saturés,
b) déshydratation des acides gras hydroxylés saturés conduisant à des acides gras mono-insaturés,
c) coupure oxydante au niveau de la double liaison des acides gras mono-insaturés, y compris dérivés nitriles insaturés, conduisant à un composé α-ω-bifonctionnel de type (ou parmi) diacide ou nitrile acide.

Le terme « acide » en particulier « acide gras hydroxylé insaturé » comme matière de départ pour le procédé de l'invention, par exemple comme défini à l'étape a) d'hydrogénation ci-dessus et pour la suite de la description, signifie et inclut systématiquement pour la suite (si on parle d' « acide » comme matière de départ) sauf indication plus spécifique (signifie et inclut) aussi bien les acides et les esters d'alcools (comme méthylique) ou les esters de polyols (comme de glycérol qui est l'huile) pour ces acides gras.

Justement, selon un mode plus particulier de l'invention, la matière « acide » de départ peut être sous forme d'ester dudit acide gras.

Dans le dernier cas de produit qui est un composé α-ω nitrile-acide, selon le procédé de la présente invention, on utilise au départ comme matière première pour la réaction d'oxydation un nitrile gras insaturé, préparé selon une première option, après l'étape b) par ammoniation (traitement à l'ammoniaque dudit acide gras).

Selon une deuxième option du procédé, ledit composé α-ω nitrile-acide peut également être préparé à partir d'un nitrile insaturé, résultant de la nitrilation de l'acide hydrogéné issu de l'étape a) conduite en même temps que l'étape de déshydratation b).

Donc, la nitrilation conduisant audit nitrile insaturé, matière première de coupure oxydante de l'étape c), peut être préparée, soit lors d'une étape séparée après l'étape b), soit en même temps que l'étape b) de déshydratation. De préférence, on fait déshydratation et ammoniation de manière simultanée.

D'une manière générale pour l'invention, le terme « ...est compris entre a et b » signifie sauf spécification contraire « ...est compris entre a et b avec a et b inclus » ou signifie la même chose que « ...varie de a à b »).

Selon une option de ce procédé, l'étape a) d'hydrogénation est conduite à une température comprise entre 70 et 180°C, de préférence entre 70 et 150°C, plus préférentiellement entre 90 et 130°C, sous une pression de H₂ comprise entre 1 et 300 bars, de préférence entre 5 et 50 bars, en présence de catalyseurs d'hydrogénation soit homogènes soit hétérogènes. Lesdits catalyseurs peuvent être des métaux nobles tels que Pt, Pd ou Rh ou des métaux de transition tels que Mo, W, Cr, Fe, Co, Ni, utilisés seuls ou en mélange éventuellement sous forme supportée sur charbon actif, sur alumine et sur silice. Parmi les catalyseurs préférés, on peut citer le nickel de Raney et/ou le palladium sur charbon actif.

Le procédé de l'invention peut utiliser comme charge, soit l'acide gras ou l'ester gras issus de l'hydrolyse ou de l'alcoolyse préalable de l'huile naturelle, soit l'huile brute elle-même. Dans ce dernier cas, il faudra prévoir une étape supplémentaire d'hydrolyse ou d'alcoolyse préalable à la coupure oxydante afin de séparer la glycérine du milieu réactionnel.

L'étape d'hydrogénation est conduite dans des conditions opérationnelles telles que l'on réalise la saturation des doubles liaisons présentes dans la charge qui contient, à la fois, les acides gras hydroxylés insaturés, des acides gras saturés, mono-insaturés ou polyinsaturés, en conservant les fonctions hydroxyles présentes.

Selon un cas particulier, l'étape a) d'hydrogénation est conduite dans des conditions opérationnelles telles que l'effluent issu de cette étape d'hydrogénation présente un indice d'iode < 5, de préférence < 3 et de façon plus préférée < 1 et un indice d'hydroxyle > 100 mg KOH/g.

L'effluent issu de cette étape d'hydrogénation présentera donc un indice d'iode (tel que défini Vol A 10 page 214 de l'encyclopédie ULLMANN) inférieur à 5, de préférence inférieur à 3 et de façon plus préféré inférieur à 1 et un indice d'hydroxyle (tel que défini Vol A 10 page 214 de l'encyclopédie ULLMANN) supérieur à 100 mg KOH/g. Les conditions opératoires seront telles que la réduction de l'indice d'hydroxyle du milieu réactionnel au terme de cette étape d'hydrogénation sera ≤ à 10 mg KOH/g.

Concernant l'étape b) de déshydratation des acides gras hydroxylés saturés, elle est conduite à une température comprise entre 100 et 300°C et en présence d'un catalyseur acide, de préférence choisi parmi : l'acide sulfurique, l'acide phosphorique, les acides sulfoniques, les alkylsulfonates, les résines acides échangeuses d'ions telles que les résines de type Amberlyst^{®}.

Concernant l'étape c) de coupure oxydante, elle est réalisée en utilisant un agent oxydant choisi parmi KMnO4, l'eau oxygénée, ozone oxydant éventuellement associé à un catalyseur (par exemple l'acide tungstique), en particulier l'ozone associée à l'oxygène.

L'invention concerne aussi bien les diacides que les α-ω aminoacides obtenus selon le procédé de l'invention.

Selon une option, le procédé de l'invention comporte une étape intermédiaire supplémentaire entre l'étape b) et l'étape c) comme définies ci-dessus, de nitrilation de la fonction acide de l'acide gras mono-insaturé, conduisant à un nitrile insaturé.

Selon encore une option particulière, le procédé de l'invention comporte une étape de nitrilation de la fonction acide de l'acide gras hydroxylé saturé issu de l'étape a) avec déshydratation concomitante (ou simultanée) conduisant à un nitrile insaturé.

Ladite étape de nitrilation peut être réalisée en phase liquide ou en phase gaz au moyen d'ammoniac à une température généralement comprise entre 150°C et 350°C et avec un catalyseur.

Plus particulièrement, ladite nitrilation est réalisée en phase liquide avec comme catalyseur un oxyde métallique qui est l'oxyde de zinc. Encore plus particulièrement, ladite nitrilation est réalisée en phase gaz avec ledit catalyseur étant par exemple supporté sur un lit fixe d'alumine dopée ou non.

Selon le procédé de l'invention, l'effluent de l'étape de nitrilation peut être soumis à l'étape c) de coupure oxydante définie ci-dessus, dont l'effluent (comprenant le composé) nitrile-acide est soumis à une hydrogénation d), comme décrit ci-dessous.

En particulier, l'étape d'hydrogénation est conduite à une température comprise entre 70 et 200°C, de préférence entre 70 et 150°C et plus préférentiellement entre 90 et 130°C, sous une pression de H₂ comprise entre 1 et 300 bars, de préférence entre 5 et 50 bars en présence de catalyseurs d'hydrogénation, soit homogènes, soit hétérogènes.

L'hydrogénation des acides gras insaturés est une réaction bien connue. On peut se référer à ce sujet au Vol A 10 de l'encyclopédie ULLMANN, pages 189, 207 à 209 et 267 à 269. Cette étape d'hydrogénation peut être conduite à une température comprise entre 70 et 150°C, de préférence entre 90 et 130°C et de manière plus préférée à environ 120°C, sous une pression de H₂ comprise entre 1 et 300 bars, de préférence entre 5 et 50 bars et de manière plus préférée à environ 20 bars. Le procédé est conduit en présence de catalyseurs d'hydrogénation, soit homogènes, soit hétérogènes, et de préférence hétérogènes. Ces catalyseurs seront par exemple des métaux nobles tels que Pt, Pd ou Rh ou des métaux de transition tels que Mo, W, Cr, Fe, Co, Ni, utilisés seuls ou en mélange. Ils pourront être déposés sur des supports tels que charbon actif, alumine et silice. Les catalyseurs préférés sont le nickel de Raney ou le palladium sur charbon actif. La quantité de catalyseur utilisé représente de 0,2 à 5% poids et de préférence de 0,4 à 2% poids de la charge traitée.

L'étape a) d'hydrogénation est conduite de préférence dans des conditions opérationnelles telles que l'effluent issu de cette étape d'hydrogénation présente un indice d'iode < 5, de préférence < 3 et de façon plus préférée < 1 et un indice d'hydroxyle > 100 mg KOH/g.

L'étape b) de déshydratation des acides gras hydroxylés saturés est conduite de manière conventionnelle à une température comprise entre 200 et 300°C en présence d'un catalyseur acide. Ce catalyseur pourra être de l'acide sulfurique, de l'acide phosphorique, les acides sulfoniques, des alkylsulfonates ou des résines acides échangeuses d'ions telles que les résines de type Amberlyst^{®} ou d'autre type.

Une abondante et ancienne littérature existe sur la déshydratation de l'huile de ricin. On peut citer notamment les brevets GB 671368, 687986, 691484, 703363 et US 2567925, ainsi que dans l'Encyclopédie KIRK-OTHMER, Vol. 8, page 526. Il est à noter que la déshydratation conduit à un mélange de deux acides gras mono-insaturés dont la double liaison est localisée, soit en « δ-n », soit en «δ-n+1 », par rapport à la fonction acide. En considérant par exemple l'acide ricinoléique, le mélange sera δ11 plus δ12 et l'acide lesquérolique δ13 plus δ14.

L'étape c) de coupure oxydante de la double liaison des acides ou nitriles gras mono-insaturés qui conduit à la formation de la fonction acide sur les deux carbones de la double liaison, est également en elle-même connue. Elle peut être réalisée en utilisant une large gamme d'agents oxydants forts.

L'étape c) de coupure oxydante peut être réalisée en utilisant par exemple un agent oxydant fort tel que KMnO₄ sous forme concentrée et à la chaleur ainsi que cela est décrit dans « Organic Chemistry » de L.G. Wade Jr., 5th Edition, Chapter 8 : Reactions of Alkenes.

La coupure oxydante peut être obtenue par une voie sulfochromique telle que décrite dans le brevet USP 2,871,247 aux colonnes 2 et 3.

L'étape c) de coupure oxydante peut être réalisée en utilisant de l'eau oxygénée comme décrit dans le brevet GB 743,491, éventuellement associée à un catalyseur. L'utilisation de H₂O₂ est également décrite dans le brevet WO 2007/039481 (Novamont).

On peut également mentionner l'ouvrage « Angew. Chem. Int. », Ed. 2000, 39, pp. 2206-2224 qui décrit la coupure oxydante de la double liaison soit avec un peracide associé à un catalyseur à base de ruthénium, soit avec H₂O₂ avec des catalyseurs à base de Mo, W ou Re.

De nombreux travaux ont été conduits sur l'utilisation de l'ozone comme agent oxydant. Il est d'ailleurs mentionné dans l'ouvrage « Angew. Chem. », cité ci-dessus, que la coupure oxydante de l'acide oléique en acides pélargonique et azélaique est la plus importante application industrielle de l'ozonolyse.

L'ozonolyse oxydante est très largement utilisée pour réaliser la coupure. Le brevet US 2,813,113 notamment décrit un procédé d'ozonolyse oxydante d'un acide gras tel que l'acide oléique qui consiste dans une première étape à traiter l'acide par de l'oxygène associé à de l'ozone pour former des ozonides, puis dans une deuxième étape à oxyder ces derniers par l'oxygène.

Dans ce type de réaction, on n'utilise pas de composés bloquant le processus d'oxydation au stade des cétones ou d'aldéhydes dans ce qui est appelé l'ozonolyse réductrice.

Ainsi, l'étape c) de coupure oxydante peut être réalisée en utilisant de l'ozone associée à l'oxygène.

L'invention a également pour objet des diacides préparés selon le procédé ci-dessus.

Lorsque le procédé de l'invention est destiné à la synthèse d'ω-amino-acide, il comportera, d'une part, une étape intermédiaire supplémentaire de nitrilation (ammoniation) de la fonction acide selon la réaction R-COOH + NH₃ → R-CN + 2 H₂O et, d'autre part, une étape finale de réduction de la fonction nitrile en fonction amine primaire. Le schéma réactionnel global est décrit plus loin.

Ainsi, dans un mode de réalisation particulier de l'invention, le procédé comporte une étape supplémentaire intermédiaire b'), entre l'étape b) et l'étape c), de nitrilation de la fonction acide de l'acide gras mono-insaturé conduisant à un nitrile insaturé.

Alternativement, le procédé peut comporter une étape a'), entre l'étape a) et l'étape b), de nitrilation de la fonction acide de l'acide gras hydroxylé saturé issu de l'étape a), avec déshydratation concomitante conduisant (suite à déshydratation) à un nitrile insaturé.

La réaction de nitrilation (ou d'ammoniation, les deux termes étant utilisés indifféremment) des acides gras est bien connue et suit le schéma réactionnel simplifié suivant :

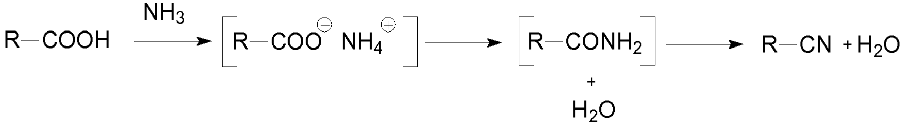

L'étape de nitrilation peut être réalisée en phase liquide (procédé batch) ou en phase gaz (procédé continu) au moyen d'ammoniac, à une température généralement comprise entre 150°C et 350°C, avec un catalyseur.

Le procédé en phase liquide est conduit à une température comprise entre 150°C environ (première phase) et 250°-300°C (deuxième phase) avec un catalyseur qui est généralement un oxyde métallique et le plus fréquemment l'oxyde de zinc.

Dans le procédé continu, la réaction a lieu en phase gazeuse à des niveaux de température élevés et généralement sur un catalyseur constitué d'un lit fixe d'alumine dopée ou non. L'acide gras est vaporisé en présence d'une large quantité d'ammoniac dont l'excès est recyclé. Le procédé peut donc être réalisé en phase gaz avec comme catalyseur un lit fixe d'alumine, dopée ou non.

Dans la version phase gazeuse, de nombreux autres catalyseurs peuvent être utilisés et il existe une abondante littérature sur ce sujet. On peut citer, par exemple, le document JP 2000-16977 qui décrit une catalyse à l'oxyde de niobium à une température de 260°C (acide stéarique) ou le document JP 2000-7637 qui décrit un procédé conduit à une température allant de 180° à 350°C, également avec un catalyseur de type oxyde de niobium. US 6,005,134 décrit une catalyse au titane et enfin JP 10-195035 décrit une catalyse à l'oxyde de zirconium dopé au fer.

Dans un mode particulier de réalisation de l'invention, l'effluent de l'étape de nitrilation est soumis à l'étape c) de coupure oxydante dont l'effluent nitrile-acide est soumis à une hydrogénation.

La réaction d'hydrogénation finale du composé nitrile-acide est réalisée dans des conditions analogues à celles décrites ci-avant pour la réaction initiale d'hydrogénation. Le nickel de Raney déposé ou non sur un support tel que la silice est le catalyseur le plus généralement adopté. L'utilisation d'autres métaux connus pour leur activité catalytique en hydrogénation a également été envisagée dans des conditions hétérogènes. On peut citer par exemple le platine, le palladium, le ruthénium ou l'iridium seuls ou en combinaison. On peut citer à ce sujet GB 1273874 qui décrit l'utilisation d'un catalyseur au ruthénium déposé sur silice.

Dans une forme de réalisation de la variante visant la synthèse d'amino-acides, on pourra dans certains cas réaliser l'étape d'ammoniation directement après l'étape d'hydrogénation initiale. En effet, dans les conditions de l'ammoniation, la déshydratation de l'acide gras hydroxylé peut intervenir spontanément, ce qui permet de supprimer une étape intermédiaire.

Dans le cas où la charge est constituée par l'huile brute, une étape supplémentaire d'hydrolyse est nécessaire et peut intervenir, soit après l'étape d'hydrogénation initiale, soit après les étapes d'hydrogénation et de déshydratation.

Les conditions opératoires de l'étape d'hydrolyse sont bien connues de l'homme de l'art. Elles sont décrites notamment dans l'Encyclopédie ULLMANN, vol. A 10, Pages 188 et 254 à 260.

Naturellement, il est parfaitement possible de procéder entre chaque étape, à une séparation des composés entrant en réaction dans l'étape suivante des composés « inutiles ».

De même, au terme du procédé, il est nécessaire de séparer les deux formes d'amino-acides ou de diacides, par exemple les C₁₁ et C₁₂ ou les C₁₃ et C₁₄ provenant de l'étape de déshydratation.

Les techniques de séparation des acides gras en mélange sont bien connues et largement décrites dans l'Encyclopédie ULLMANN, vol. A 10, pages 260 à 267. Elles sont fondées essentiellement sur la distillation et la cristallisation et leurs diverses variantes. Le choix de la technique dépendra de la différence des températures de changement d'état des constituants du mélange, liquide-gaz (distillation) ou liquide-solide (cristallisation). On peut également envisager des extractions liquide-liquide utilisant des solvants adaptés ou des séparations chromatographiques.

Dans une variante de mise en oeuvre du procédé de l'invention, on peut envisager d'enrichir le milieu réactionnel en l'un des amino-acides ou diacides ciblés, en y ajoutant en cours de processus, soit après l'étape de déshydrogénation initiale, soit après l'étape de déshydratation, un acide gras comportant une seule insaturation oléfinique en position convenable δ11, δ12, δ13 ou δ14. A titre d'exemples, on pourra ajouter au milieu, si la cible principale est l'amino-acide en C₁₁, de l'acide vaccénique et/ou de l'acide gondoïque. De même, on pourra ajouter au milieu de l'acide érucique pour augmenter la proportion de diacide C₁₃ ou nitrile-acide C₁₃.

Le procédé est particulièrement intéressant pour des charges contenant les acides densipolique et auricolique en mélange ou non avec les acides ricinoléique et lesquérolique car, après hydrogénation, on obtient des acides saturés de même longueur de chaîne C₁₈ ou C₂₀.

Dans la mise en oeuvre du procédé de l'invention, on choisira de préférence des charges riches en acide ricinoléique ou densipolique conduisant à des nitrile-acides en C₁₁ ou C₁₂ conduisant aux ω-amino-acides correspondants et des charges riches en acide lesquérolique ou auricolique conduisant aux diacides en C₁₃ ou C₁₄.

Le procédé de l'invention permet de synthétiser selon la variante mise en oeuvre, soit un diacide gras, soit un amino-acide gras comportant de 11 à 14 atomes de carbone par molécule et cela en fonction de la composition de la charge traitée.

Les schémas réactionnels du procédé sont illustrés ci-dessous en prenant comme exemple l'acide ricinoléique, lequel est le plus courant des acides gras insaturés hydroxylés. Si la charge traitée est sous forme ester, les schémas réactionnels ci-dessous seront identiques sauf à remplacer le H de la fonction acide par un radical alkyle (ester simple d'alcool tel que méthylique par exemple) ou par un radical comportant 2 ou 3 atomes de carbone porteurs respectivement de 1 ou 2 fonctions alcools ou esters (esters de polyol tel que le glycérol par exemple).

### Variante diacide

### CH₃-(CH₂)₅-CHOH-CH₂-CH=CH-(CH₂)₇-COOH + H₂ → CH₃-(CH₂)₅-CHOH-(CH₂)₁₀-COOH

La déshydratation conduit à un mélange d'acides gras insaturés selon CH₃-(CH₂)₅CHOH-(CH₂)₁₀-COOH → CH₃-(CH₂)₄-CH=CH-(CH₂)₁₀-COOH + H₂O ou CH₃-(CH₂)₅CHOH-(CH₂)₁₀-COOH → CH₃-(CH₂)₅-CH=CH-(CH₂)₉-COOH + H₂O

La coupure oxydante conduit donc à un mélange de diacides CH₃-(CH₂)₄-CH=CH-(CH₂)₁₀-COOH (+ Oxy.) → HOOC-(CH₂)₁₀-COOH + CH₃-(CH₂)₄-COOH et CH₃-(CH₂)₅-CH=CH-(CH₂)₉-COOH (+ Oxy.) → HOOC-(CH₂)₉-COOH + CH₃-(CH₂)₅-COOH

### Variante amino-acide

### CH₃-(CH₂)₅-CHOH-CH₂-CH=CH-(CH₂)₇-COOH + H₂ → CH₃-(CH₂)₅-CHOH-(CH₂)₁₀-COOH

La déshydratation conduit à un mélange d'acides gras insaturés selon CH₃-(CH₂)₅-CHOH-(CH₂)₁₀-COOH CH₃-(CH₂)₄-CH=CH-(CH₂)₁₀-COOH + H₂O ou CH₃-(CH₂)₅-CHOH-(CH₂)₁₀-COOH CH₃-(CH₂)₅-CH=CH-(CH₂)₉-COOH + H₂O

La nitrilation conduit aux nitriles gras selon CH₃-(CH₂)₄-CH=CH-(CH₂)₁₀-COOH + NH₃ → CH₃-(CH₂)₄-CH=CH-(CH₂)₁₀-CN + 2H₂O ou CH₃-(CH₂)₅-CH=CH-(CH₂)₉-COOH + NH₃ → CH₃-(CH₂)₅-CH=CH-(CH₂)₉-CN + 2H₂O

La coupure oxydante conduit donc à un mélange de nitrile-acides CH₃-(CH₂)₄-CH=CH-(CH₂)₁₀-CN (+ Oxy.) → HOOC -(CH₂)₁₀-CN + CH₃-(CH₂)₄-COOH et CH₃-(CH₂)₅-CH=CH-(CH₂)₉-CN (+ Oxy.) → HOOC -(CH₂)₉-CN + CH₃-(CH₂)₅-COOH

Enfin, l'hydrogénation des nitrile-acides conduit aux amino-acides selon HOOC -(CH₂)₁₀-CN + 2H₂ → HOOC -(CH₂)₁₀-CH₂NH₂ et HOOC -(CH₂)₉-CN + 2H₂ → HOOC -(CH₂)₉-CH₂NH₂

L'étape de nitrilation des acides gras intervient après l'étape d'hydrogénation. Elle peut suivre l'étape de déshydratation mais, dans certains cas, les conditions de la nitrilation permettront la déshydratation concomitante de l'acide gras hydroxylé saturé, ce qui sera particulièrement avantageux.

L'invention a également pour objet des ω-amino-acides préparés selon le procédé ci-dessus.

Sur un plan pratique, le procédé pourra de préférence comprendre les enchaînement d'étapes suivantes, en partant d'une charge d'origine naturelle contenant des acides gras hydroxylés insaturés sous forme d'ester (d'alcool ou de polyol comme le glycérol ou glycérine) et en particulier d'huile hydroxylée d'acide gras correspondant (l'huile étant un ester de glycérol) avec lesdites étapes étant :
i) hydrogénation de l'ester hydroxylé, en particulier de l'huile correspondante
ii) déshydratation de l'ester hydroxylé hydrogéné, en particulier de l'huile correspondante
iii) hydrolyse de l'ester hydrogéné (et déshydraté), en particulier de l'huile correspondante pour obtenir l'acide gras insaturé correspondant et de l'alcool ou du polyol, en particulier de la glycérine
iv) séparation de l'alcool ou du polyol, en particulier de la glycérine (ou glycérol) et éventuellement séparation des isomères d'acide gras insaturés (par exemple séparation du 11-octadécénoique du 12-octadécénoique)
v) en option (seulement dans le cas de nitrile) ammoniation de l'acide (gras) insaturé pour obtenir le nitrile insaturé correspondant
vi) coupure oxydante de l'acide gras ou nitrile insaturé (en fonction du cas)
vii) en option, séparation des produits de coupure et des acides légers formés (par exemple du 11- nitrile acide du 12-nitrile acide dans le cas du nitrile)
viii) en option (cas de nitrile) hydrogénation du nitrile-acide pour former l'amino-acide correspondant
ix) séparation et purification du diacide formé ou de l'amino-acide formé en fonction du cas.

Selon une autre option plus spécifique dudit procédé où l'objectif est d'abord un nitrile acide et puis un aminoacide correspondant, ledit acide ω-fonctionnalisé est donc un amino-acide et ladite charge d'origine naturelle contient des acides gras insaturés hydroxylés sous forme d'ester (d'alcool ou de polyol comme le glycérol ou glycérine), en particulier d'une huile hydroxylée (ester de glycérol) et ledit procédé comprend l'enchaînement des étapes suivantes :
i) hydrogénation de l'ester hydroxylé, en particulier de l'huile correspondante
ii) hydrolyse de l'ester hydrogéné, en particulier de l'huile correspondante, pour obtenir l'acide gras saturé correspondant (par exemple l'acide 12-hydroxy stéarique) et de l'alcool ou du polyol, en particulier de la glycérine
iii) séparation de l'alcool ou du polyol, en particulier de la glycérine avec éventuellement séparation des acides gras saturés des acides gras hydroxylés
iv) ammoniation de l'acide saturé hydroxylé, avec déshydratation simultanée, pour obtenir le nitrile insaturé correspondant
v) éventuellement (en option), séparation des isomères de nitriles insaturés
vi) coupure oxydante du nitrile insaturé
vii) en option, séparation des produits de coupure nitrile-acides et des acides légers formés
viii) hydrogénation du nitrile-acide pour obtenir l'amino-acide correspondant
ix) séparation et purification de l'amino-acide obtenu.

Dans le cas où l'objectif est un nitrile acide, puis un amino-acide, à partir d'une source d'huile végétale peu concentrée en acide hydroxylé, on peut prévoir un enchaînement légèrement différent :
i) alcoolyse (par exemple ou en particulier méthanolyse) de l'huile végétale, éventuellement concomitante à son extraction de la graine
ii) extraction d'une fraction riche en ester d'acide hydroxylé
iii) hydrogénation de l'ester hydroxylé
iv) en option, hydrolyse de l'ester hydrogéné pour obtenir l'acide gras correspondant (par exemple production de l'acide 12-hydroxystéarique ou 14-hydroxyeicosanoique)
v) ammoniation de l'acide-saturé hydroxylé avec déshydratation simultanée (y compris sur ester d'étape iii) ou sur acide d'étape iv)) pour obtenir le nitrile insaturé
vi) éventuellement, séparation des isomères de nitriles insaturés
vii) coupure oxydante du nitrile insaturé
viii) en option, séparation des produits de coupure nitrile-acides (à 11 et 12 carbones par exemple) et des acides légers formés
ix) hydrogénation du nitrile-acide
x) séparation et purification de l'amino-acide.

Les exemples qui suivent illustrent de manière encore plus détaillée la présente invention, sans aucune limitation, par ces exemples, de sa couverture.

### Partie expérimentale

### Exemple 1 : Production d'huile de Lesquerella

Le mode opératoire sur 25 kg de graines est le suivant :
1. Floconnage de la graine de lesquerella fraîche sur aplatisseur à rouleaux lisses.
2. Les flocons sont ensuite séchés 16 h à 100°C.
3. Les flocons sont introduits dans une colonne de percolation.
4. Le mélange méthanol/hexane (50/50 en poids) est alors circulé sur le lit pendant 30 minutes à 40°C.
5. Le miscella est ensuite soutiré et le lit de flocons est ensuite lavé par 5 lavages successifs avec le mélange méthanol/hexane à 40°C (5 minutes par lavage).
6. Le miscella est alors évaporé sous vide à 90°C sous 20 mbars pendant 5 min.
7. L'huile et les gommes sont séparées par centrifugation. Le rendement en huile est calculé sur la base de la masse d'huile obtenue versus la masse d'huile théorique attendue.
8. L'huile est ensuite lavée jusqu'à neutralité par ajout d'eau chaude et centrifugation puis elle est séchée sous vide à 90°C et 20 mbars pendant 5 min. L'indice d'acide et la composition de cette huile sont alors mesurés.

**Tableau A : Bilan massique du procédé de trituration non réactive de la graine de lesquerella en présence d'un mélange méthanol/hexane**

| **Conditions** | **ESSAI 10-E47** |
|---|---|
| Aplatissage rouleaux lisses serrés | Oui |
| Séchage 100°C, 16 h | Oui |
| Epaisseur flocon, mm | 0,16 à 0,18 |
| Rapport massique Solvants / Graine | 2 |

| **Bilan Essai** | |
|---|---|
| Rendement (Rdt) en extrait sec, % (1) | 107,8 |
| Déphasage | Non |
| Rdt Huile, % | **107,8** |
| Potentiel Huile dans le tourteau, % | 2,4 |
| (Teneur MG tourteau, %) | (0,9) |
| Rdt en insolubles dans l'huile extraite par centrifugation, % | **0,0** |
| Perte en huile (valeur calculée), % (2) | **-10,2** |

| | |
|---|---|
| (1) Le rendement en extrait sec est le rapport fois 100 de l'extrait sec obtenu après évaporation du miscella sur la somme des quantités théoriques d'huile théorique (2) Perte Huile = 100 - Rdt Huile - Pot. Huile Tourteau | |

### Commentaires :

- En présence d'un mélange méthanol/hexane, le rendement d'extraction est du même ordre qu'avec du méthanol seul (≈ 108%) alors que la température d'extraction a été ramenée à 40°C afin d'éviter l'ébullition de l'hexane ;
- la teneur en insoluble est nulle, semblant indiquer que s'il y a eu extraction de gommes, ces dernières sont partiellement liposolubles (< 10% dans l'huile) ;
- le tourteau reste relativement bien épuisé (0,9%) ;
- la teneur en phospholipides de l'huile est de 1,3% ;
- sur le plan qualitatif, l'huile extraite est très acide (IA > 11). Il est fort probable que les gommes solubilisées présentent une acidité significative. Par ailleurs, on retrouve la teneur attendue en acide lesquerolique (52%).

**Tableau 1 : Analyse de l'huile extraite avec un mélange méthanol/hexane**

| **Critères** | **Méthode** | **Essai 10-E47 Huile** |
|---|---|---|
| Indice d'acide (mg KOH/g) | EN 14104 | **11,2** |
| Profil en acides gras | EN14105 | |
| Palmitique (C16:0) | | 1,6 |
| Palmitoléique (C16:1) | | 0,9 |
| Stéarique (C18:0) | | 2,3 |
| Oléique (C18:1) | | 18,8 |
| Ricinoléique (C18:1-OH) | | 0,5 |
| Linoléique (C18:2) | | 10,1 |
| Densipoléique (18:2-OH) | | 0,2 |
| Linolénique et Arachidique | | 11,6(1) |
| Eicosénoique (C20:1) | | 0,9 |
| **Lesquerolique (C20:1-OH)** | | **52,1** |
| Auricolique (20:2-OH) | | 2,6 |
| Phospholipides (%) | Interne | **1,3 (2)** |
| Rendement corrigé en huile (3), % | - | **106,5** |

| | | |
|---|---|---|
| (1) Les deux pics sont co-élués. L'acide linolénique est majoritaire (2) Valeur calculée ; % phospholipides = % phosphore x 26 (3) Rdt corrigé = Rdt Huile d'extraction (tableau 1) - % phospholipides | | |

L'huile ainsi obtenue est ensuite raffinée par neutralisation à la soude et dégommage à l'acide phosphorique dilué de façon à éliminer les phospholipides. Finalement, l'huile est séchée sous vide. L'huile obtenue a les caractéristiques suivantes :
Indice d'acide : 0,5 mg KOH/g
Indice de saponification : 175 mg KOH/g
Indice d'hydroxyle : 100 mg KOH/g
Indice d'iode : 95 g I₂/100 g
Teneur en acide lesquerolique : 52%
Teneur en phosphore : 10 ppm
Teneur en eau et volatils : 0,1% pds
Teneur en cendres : 0,1% pds.

### Exemple 2 : Préparation des acides gras hydrogénés de Lesquerella

Dans un premier temps, on procède à une transestérification de l'huile de Lesquerella, puis à une hydrogénation et finalement à une hydrolyse. Une étape d'extraction après la transestérification permet d'enrichir le produit en ester d'acide lesquerolique.

La méthanolyse de l'huile de lesquerella est effectuée avec un rapport molaire Méthanol/huile de 6 (soit deux fois la quantité stoechiométrique). Le catalyseur utilisé est le méthylate de sodium à une teneur de 0,5% poids et la température de la réaction est de 60°C. Les constituants sont mélangés sous forte agitation pendant 30 minutes. Après méthanolyse (transestérification) et élimination de la glycérine par décantation, les esters sont purifiés par lavage à l'eau et séchage sous vide. Les spécifications des esters méthyliques sont les suivantes :
Indice d'acide : 0,5 mg KOH/g
Indice de saponification : 175 mg KOH/g
Indice d'Iode : 95 g I2/100 g
Teneur en glycérides résiduels (analyse par CPG) : 1,9% pds
Teneur en acide Lesquerolique : 52%

Les esters méthyliques en mélange, obtenus à l'étape précédente, sont hydrogénés en autoclave (en plusieurs fois du fait de la taille de l'autoclave). On utilise un catalyseur de type nickel de Raney fourni par Johnson Matthey à une teneur de 0,5% en poids. La température d'hydrogénation est de 150°C, sous une pression d'hydrogène de 8 bars. Cette étape conduit à un produit ayant un indice d'iode de 3 g I2/100 g et un indice d'hydroxyle de 93 mg KOH/g.

On procède finalement à une étape de saponification par ajout de soude au mélange d'esters, puis à une étape d'acidification à l'acide sulfurique. Le mélange résultant est lavé à l'eau, décanté et séché sous vide. Le mélange d'acide gras obtenu a les caractéristiques suivantes :
Indice d'acide de 180 mg KOH/g
Indice d'Hydroxyle de 90 mg KOH/g
Indice d'iode de 3 g I₂/100 g

La teneur en acide 14-hydroxyeicosanoique est de 50%.

### Exemple 3 : Préparation d'un mélange enrichi en acide 14-Hydroxyeicosanoique

Le mélange d'esters issus de l'étape de transestérification comme dans de l'exemple 2, mais à partir d'une huile de Lesquerella commerciale, est soumis à une étape d'extraction liquide-liquide avec un mélange Méthanol/hexane. Dans la réalisation pratique de l'exemple, le méthanol contient 5% en poids d'eau. Les acides gras non hydroxylés sont plus compatibles avec la phase hexane, alors que les acides gras hydroxylés comme l'acide lesquerolique sont plus compatibles avec la phase méthanolique.

Dans cet exemple, l'hexane a été utilisé en tant que solvant apolaire. Pour rappel, le solvant polaire est constitué de méthanol hydraté. On procède à une succession d'étapes d'appauvrissement et d'enrichissement.
1. 5 g (ester méthylique d'huile de Lesquerella) + 30 mL de solvant apolaire + 15 mL solvant polaire sont agités pendant 5 minutes dans une ampoule à décanter et donnent une phase lourde PL1 + phase légère pl1.
2. La phase légère pl1 est reprise avec 15 mL de solvant polaire et donne à nouveau une phase lourde PL2 et une phase légère pl2.
3. La phase lourde PL1 et la phase lourde PL2 sont reprises avec 30 mL de solvant apolaire et donnent à nouveau une phase lourde PL3 et une phase légère pl3.
4. La phase lourde PL3 est reprise avec 30 mL de solvant apolaire pour donner une phase lourde PL4 et une légère pl4.

On concentre ensuite, par évaporation des solvants, les fractions récupérées.
1. La phase lourde PL4 donne la fraction polaire.
2. Les phases légères pl2 + pl3 + pl4 sont combinées pour donner la fraction apolaire.

**Tableau n°2 : Bilan analytique des esters issus de l'extraction**

| | Matière première | Phase Lourde | Phase Légère |
|---|---|---|---|
| Solvant polaire | | Méthanol 95% | Méthanol 95% |
| Solvant apolaire | | Hexane | Hexane |
| Rdt massique, % | | 16,5 | 83,5 |
| Rdt extraction Lesquerolate de methyle, % | | 25,4 | 74,8 |
| Indice d'acide | 0,72 | nd | 1,12 |
| Me C16:1 (%) | 0,5 | 0,1 | 0,6 |
| Me C16 (%) | 1,3 | 0,1 | 1,6 |
| Me C18 :2 (%) | 9,1 | 0,6 | 11,7 |
| Me C18 :1 (%) | 23,5 | 0,3 | 27,5 |
| Me C18 :0 (%) | 1,8 | 0,1 | 2,2 |
| Me C20:0 (%) | 0,9 | 0,0 | 1,1 |
| Me C20:1 (%) | 1,0 | 0,0 | 0,7 |
| Me C18 :1-OH (%) | 0,3 | 0,2 | 0,3 |
| Me C20 :1-OH (%) | **60,3** | **92,9** | **54,0** |
| MonoGlycéride (%) | **1,7** | **5,8** | **0,8** |
| DiGlycéride (%) | 0,1 | 0,0 | 0,1 |
| TriGlycéride (%) | 0,0 | 0,0 | 0,0 |

Sur la fraction enrichie en acide lesquerolique, on procède, comme dans l'exemple précédent, à une hydrogénation et à une hydrolyse pour obtenir un mélange riche en acide 14-hydroxyeicosanoique.

Les caractéristiques du mélange obtenu sont :
Indice d'acide de 1 mg KOH/g
Indice d'hydroxyle de 145 mg KOH/g
Indice d'iode de 3 g I2/ 100 g
Teneur en acide 14-hydroxyeicosanoique de 89%.

### Exemple 4 : Préparation de l'acide 12-hydroxystéarique

Dans cet exemple, on procède comme dans l'exemple 6.1 essai 09-E08 de la demande de brevet WO 2010/076527 par trituration réactive de graines de ricin. Le mélange d'esters méthyliques est ensuite hydrogéné et hydrolysé. Comme dans l'exemple 2, on obtient un mélange riche en acide 12-hydroxystéarique.

Après l'étape de trituration réactive, on obtient un mélange d'esters méthyliques ayant un indice d'acide de 0,46 mg KOH/g, contenant 0,85% d'ester d'acide palmitique, 2,9% d'ester d'acide linoléique, 3,46% d'ester d'acide oléique, 0,96% d'ester d'acide stéarique, 90,65% d'ester d'acide ricinoléique. Le mélange contient aussi 1,19% de monoglycérides.

Le mélange d'esters est alors hydrogéné en présence d'un catalyseur Nickel de Raney (1% pds), sous une pression de 20 Bars d'hydrogène et à une température de 130°C. La réaction est effectuée en 2 heures. Le produit final est ensuite saponifié à la soude et acidifié à l'acide sulfurique. Après lavage, on obtient un mélange riche en acide 12-hydroxystéarique contenant 0,9% d'acide palmitique, 0,3% d'acide linoléique, 0,4% d'acide oléique, 7,8% d'acide stéarique, 90% d'acide 12-hydroxystéarique.

### Exemple 5 : Préparation du nitrile isooléique (cette expression est utilisée pour indiquer un mélange d'isomères insaturés à 18 atomes de Carbone) à partir de l'acide 12-hydroxystéarique commercial

Dans cet exemple, on utilise un acide 12-hydroxystéarique commercial fourni par la société MOSSELMAN. Ce lot a les spécifications suivantes :
Indice d'acide : 177 mg KOH/g
Indice de saponification : 182,5 mg KOH/g
Indice d'iode : 2,3 g I2/ 100 g
Indice d'hydroxyle : 162,7 mg KOH/g.

Cette étape consiste à faire réagir l'ammoniac sur l'acide gras à température élevée en présence d'un catalyseur (oxyde de zinc) afin d'obtenir le nitrile correspondant. Le mécanisme réactionnel est probablement une conversion de l'acide en sel d'ammonium, puis en amide et finalement en nitrile. Du fait des conditions extrêmes de cette réaction, l'acide hydroxylé se déshydrate simultanément.

Pour effectuer cette réaction, on utilise un montage constitué d'un réacteur chauffé par un chauffe ballon, surmonté par un condenseur (déflegmateur) qui renvoie dans le réacteur les composés lourds et laisse passer l'eau issue de la réaction ainsi que l'excès d'ammoniac. Le réacteur est alimenté par de l'ammoniac gazeux. La bouteille d'ammoniac est posée sur une balance pour pouvoir contrôler la quantité utilisée.

On charge 250 g d'acide 12-hydroxystéarique dans le réacteur. On ajoute 0,16 g de catalyseur oxyde de zinc. La température du mélange est amenée jusqu'à la fusion de l'acide (82°C), puis sous agitation à 205°C. A cette température, l'ammoniac est ajouté dans le réacteur en augmentant progressivement le débit jusque 0,417 litres/minute.kg. Ensuite, on augmente la température du réacteur jusqu'à 300°C. La température du déflegmateur est maintenue à 130°C.

La température du réacteur est maintenue à 300°C jusqu'à ce que l'on atteigne un indice d'acide inférieur à 0,1 mg KOH/g. Le suivi de l'avancement de la réaction est effectué en mesurant l'indice d'acide et la quantité d'eau condensée en sortie de réacteur. Dans le cas présent, l'ammoniac en excès n'est pas recyclé, mais il peut l'être industriellement. Après 10 heures de réaction et 72 g d'ammoniac ajoutés, la réaction est arrêtée. On récupère 189,5 g de produit (mais une part des pertes est liée aux nombreux échantillonnages au cours de la synthèse). Le produit brut est ensuite distillé sous une pression réduite à 16 mBars avec une colonne Vigreux, ce qui revient à éliminer les produits lourds. La distillation est effectuée à une température de 216 à 289°C dans le bouilleur et 205 à 219°C en tête de colonne de distillation. L'ensemble du produit est passé en 20 minutes. On récupère 159 g de distillat et 15,3 g de fraction de tête pour 13 g de culot de distillation (fraction lourde), soit un rendement de distillation de 92%.

Le produit issu de cette réaction est analysé par chromatographie en phase gaz, sur chromatographe HP5890 série II, ave une colonne HP5 (30 m).

Le produit brut obtenu contient 0,9% en poids (pds) de nitrile palmitique, 86,3% pds de nitrile isooléique, 9,1% pds de nitrile stéarique, 0,4% pds de nitrile linoléique, 0,3% pds de nitrile eicosénoique et 0,3% pds de nitrile eicosanoique.

### Exemple 6 : Préparation du nitrile à partir de l'acide 12-hydroxystéarique obtenu selon l'exemple 4

On procède comme dans l'exemple précédent, mais à partir de l'acide 12-hydroxystéarique obtenu selon l'exemple 4. On obtient un mélange riche en Nitrile isooléique contenant 0,9% de nitrile palmitique, 0,2% de nitrile linoléique, 8,0% d'acide stéarique et 90,4% de nitrile isooléique.

### Exemple 7 : Préparation de l'acide iso-oléique obtenu par déshydratation de l'acide 12-hydroxystéarique et préparation du nitrile correspondant

On utilise pour cet exemple l'acide 12-hydroxystéarique commercial de l'exemple 5. La réaction de déshydratation est suivie en contrôlant l'indice d'iode et l'indice d'hydroxyle. Pour effectuer cette réaction, une résine acide Amberlyst^{®} 15 est utilisée comme catalyseur à une concentration de 5% pds. A une température de réaction de 120°C et sous un vide poussé de 400 mBars pour éliminer l'eau produite par la réaction, un produit ayant un indice d'iode de 68 et un indice d'hydroxyle de 6 a été obtenu.

Dans les mêmes conditions et à une température de 150°C, un indice d'hydroxyle nul est obtenu avec un indice d'iode de 75.

Le produit brut obtenu contient 1,0% pds d'acide palmitique, 85,2% pds d'acide isooléique, 9,2% pds d'acide stéarique, 0,3% pds d'acide linoléique, 0,3% pds d'acide eicosénoique et 0,3% pds d'acide eicosanoique.

### Exemple 7 bis : Préparation de l'acide iso-oléique par déshydratation de l'acide 12-hydroxystéarique et préparation du nitrile correspondant

On utilise pour cet exemple l'acide 12-hydroxystéarique commercial de l'exemple 5. La réaction de déshydratation est suivie en contrôlant l'indice d'iode et l'indice d'hydroxyle. Pour effectuer cette réaction, un catalyseur silice-alumine contenant 10% d'alumine (ayant une surface spécifique de 406 m²/g) est utilisé comme catalyseur à une concentration de 10% pds. A une température de réaction de 180°C, sous un vide partiel pour éliminer l'eau produite par la réaction, un produit ayant un indice d'iode de 72 et un indice d'hydroxyle de 6 est obtenu.

### Exemple 7 ter : Préparation de l'acide iso-oléique par déshydratation de l'acide 12-hydroxystéarique

On utilise pour cet exemple le mélange d'esters méthyliques hydrogénés produit selon l'exemple 4. La réaction de déshydratation est suivie en contrôlant l'indice d'iode et l'indice d'hydroxyle. Pour effectuer cette réaction, on utilise de l'acide sulfurique (0,1%) et une argile activée (1,0% pds) comme catalyseur. La réaction a lieu à une température de 180°C pour une durée de 3 heures et sous un vide partiel pour éliminer l'eau produite par la réaction. Après réaction, le produit est lavé et séché pour éliminer le catalyseur. Un produit ayant un indice d'iode de 55 et un indice d'hydroxyle de 2 est obtenu.

### Exemple 8: Préparation du nitrile iso-eicosénoique à partir de l'acide 14-hydroxyeicosanoique issu de l'exemple 2

On procède comme dans l'exemple 5 à partir de l'acide 14-hydroxyeicosanoique issu de l'exemple 2 et on obtient un mélange de nitriles contenant 51% de nitrile iso-eicosenoique. L'augmentation de la teneur en nitrile C20 :1 par rapport à l'acide lesquerolique peut s'expliquer par la forte réactivité des acides polyinsaturés au cours de la conversion en nitriles, ceux-ci conduisant à des produits lourds qui sont éliminés dans le culot de distillation.

### Exemple 9: Préparation du nitrile isoeicosénoique à partir de l'acide 14-hydroxyeicosanoique issu de l'exemple 3

On procède comme dans l'exemple 8 précédent avec le mélange riche en acide 14-hydroxyeicosanoique de l'exemple 3. On obtient un mélange de nitriles contenant 90% de nitrile iso-eicosenoique.

### Exemple 10 : Coupure oxydante à l'eau oxygénée du nitrile isooléique issu de l'exemple 5

25 g de produit obtenu, tel qu'à l'exemple 5, sont mélangés à 250 mg d'acide tungstique dans un réacteur à double enveloppe. Sous agitation mécanique, la température est régulée à 70°C et environ 7 grammes d'eau oxygénée (H₂O₂) à 70% pds sont ajoutés goutte à goutte en environ 7 minutes. Après 2 heures de temps de réaction, la phase aqueuse est éliminée par décantation. 250 mg d'acide tungstique sont à nouveau ajoutés à la phase organique et à nouveau 7 g d'eau oxygénée à 70% sont ajoutés au goutte à goutte comme précédemment. Cette opération est répétée après 4 h, 22 h, 24 h et 26 h de temps de réaction, avec une durée totale de l'essai de 28 heures et 42 g d'H₂O₂ ajoutés.

Après séparation de la dernière fraction de phase aqueuse, la phase organique est lavée à l'eau, puis séchée sous vide et analysée par chromatographie.

La composition du mélange obtenu indique un rendement en acide hexanoique de 10,4% mol, en acide heptanoique de 12,3% pds, en acide 10-cyanodécanoique de 13,1% pds, en acide 11-cyanoundecanoique de 12,1% pds.

Cette distribution confirme que la réaction de déshydratation de l'acide 12-Hydroxystéarique qui s'est produite au cours de la formation du nitrile, a conduit aux deux isomères insaturés C18:1 delta-11 et C18:1 delta-12.

### Exemple 11 : Coupure oxydante à l'eau oxygénée de l'acide isooléique obtenu à l'exemple 7

On procède comme dans l'exemple 10, mais avec le mélange d'acide riche en acide isooléique issu de l'exemple 7.

Le rendement est de 8% en acide heptanoique et de 7,1% en acide hexanoique. Ces deux acides sont les plus faciles à analyser et caractérisent la coupure oxydante du milieu. Après estérification du mélange obtenu, la quantification des diacides produits indique un rendement en diacides de 8,5% en undécanedoique et de 7,5% en dodécanedioique.

### Exemple 12 : Ozonolyse du nitrile iso-ecosénoique obtenu à l'exemple 8 et hydrogénation du nitrile acide (cyanoacide) obtenu, en amino acide correspondant

Cet exemple illustre la coupure oxydante du nitrile insaturé issu de l'exemple 8 par ozonolyse pour former les nitriles-acides de formule CN-(CH₂)₁₁-COOH et CN-(CH₂)₁₂-COOH.

De l'ozone obtenu par un générateur d'ozone Ozonia est bullée dans 50 grammes d'un nitrile obtenu conformément à l'exemple 8. On produit une quantité d'ozone de 50 g/h, à une concentration de 6% dans de l'oxygène pur. L'ensemble de l'installation est en verre. Pendant cette étape, d'une durée de 4 heures, la température de la solution est maintenue inférieure à 30°C. Pour effectuer la conversion de l'ozonide en acide-nitrile, on commence par élever la température à environ 60°C. Quand la réaction de décomposition de l'ozonide démarre, elle s'accompagne d'une élévation de la température. On ajoute en continu un flux d'oxygène pour maintenir la température et pour oxyder directement les produits issus de la décomposition de l'ozonide. On procède en 4 heures pour limiter la formation de produits de dégradation. Il est important de maintenir la température de réaction légèrement au-dessus de la température de décomposition de l'ozonide au cours de cette étape. Une température de 95°C est utilisée dans cet exemple.

On obtient un rendement de 91% molaire en un mélange d'acide 10-cyanodécanoique et d'acide 11-cyanoundecanoique.

15 g de nitrile acide sont dissous dans 160 g d'éthanol. La solution est placée dans un autoclave agité, avec 3 g de catalyseur nickel de Raney ; on ajoute alors 15 g d'ammoniac et une pression de 110 bars d'hydrogène. La température est élevée à 100°C et la pression augmente jusqu'à 139 bars. Les conditions sont maintenues pendant 4 heures. L'autoclave est refroidi et le contenu est filtré pour récupérer le catalyseur. 50 g d'eau sont alors ajoutés et l'alcool est distillé. La solution résultante est titrée avec de l'acide chlorhydrique dilué et le mélange d'aminoacides est filtré, lavé et traité sous reflux d'acétone et séché.

### Exemple 13 : Ozonolyse du nitrile iso-ecosénoique obtenu à l'exemple 9

On procède comme à l'exemple 12. A l'issu de l'étape d'ozonolyse, le mélange réactionnel est constitué majoritairement d'acide heptanoique et d'acide hexanoique, ainsi que de cyanoacides. Les acides heptanoiques et hexanoiques sont éliminés par lavage à l'eau chaude. Les cyanoacides sont récupérés par extraction au cyclohexane à basse température et recristallisation. Le mélange de cyanoacides est alors hydrogéné comme dans l'exemple 12.

## Revendications

1. Procédé de synthèse d'acides ω-fonctionnalisés, **caractérisé en ce que** lesdits acides sont de formule R-(CH₂)ₙ-COOH, dans laquelle R représente COOH ou NH₂CH₂ et n un entier compris entre 9 et 12, ils sont obtenus à partir d'une charge d'origine naturelle contenant des acides gras hydroxylés insaturés sous forme acide, ester ou ester de polyol comportant au moins 18 atomes de carbone par molécule et **en ce que** ledit procédé comporte les étapes suivantes :
a) hydrogénation des acides gras hydroxylés insaturés conduisant à des acides gras hydroxylés saturés,
b) déshydratation des acides gras hydroxylés saturés conduisant à des acides gras mono-insaturés,
c) coupure oxydante au niveau de la double liaison des acides gras mono-insaturés, conduisant à un composé α-ω-bifonctionnel parmi diacide ou nitrile-acide.

2. Procédé selon la revendication 1, **caractérisé en ce que** l'étape a) d'hydrogénation est conduite à une température comprise entre 70 et 180°C, de préférence 70 et 150°C, plus préférentiellement entre 90 et 130°C, sous une pression de H₂ comprise entre 1 et 300 bars, de préférence entre 5 et 50 bars en présence de catalyseurs d'hydrogénation, soit homogènes, soit hétérogènes.

3. Procédé selon la revendication 2, **caractérisé en ce que** lesdits catalyseurs sont des métaux nobles tels que Pt, Pd ou Rh ou des métaux de transition tels que Mo,W, Cr, Fe, Co, Ni, utilisés seuls ou en mélange éventuellement sous forme supportée sur charbon actif, sur alumine et sur silice.

4. Procédé selon la revendication 2 ou 3, **caractérisé en ce que** lesdits catalyseurs sont choisis parmi le nickel de Raney et/ou le palladium sur charbon actif.

5. Procédé selon l'une des revendications 1 à 4, **caractérisé en ce que** l'étape a) d'hydrogénation est conduite dans des conditions opérationnelles telles que l'effluent issu de cette étape d'hydrogénation présente un indice d'iode < 5, de préférence < 3 et de façon plus préférée < 1 et un indice d'hydroxyle > 100 mg KOH/g.

6. Procédé selon l'une des revendications 1 à 5, **caractérisé en ce que** l'étape b) de déshydratation des acides gras hydroxylés saturés est conduite à une température comprise entre 100 et 300°C et en présence d'un catalyseur acide, de préférence choisi parmi : l'acide sulfurique, l'acide phosphorique, les acides sulfoniques, les alkylsulfonates ou les résines acides échangeuses d'ions.

7. Procédé selon l'une des revendications 1 à 6, **caractérisé en ce que** l'étape c) de coupure oxydante est réalisée en utilisant un agent oxydant choisi parmi KMnO4, l'eau oxygénée, ozone oxydant éventuellement associé à un catalyseur tel que l'acide tungstique, en particulier l'ozone associée à l'oxygène.

8. Procédé selon l'une des revendications 1 à 7, **caractérisé en ce qu'**il comporte une étape intermédiaire supplémentaire entre l'étape b) et l'étape c) de nitrilation de la fonction acide de l'acide gras mono-insaturé conduisant à un nitrile insaturé.

9. Procédé selon l'une des revendications 1 à 7, **caractérisé en ce qu'**il comporte une étape de nitrilation de la fonction acide de l'acide gras hydroxylé saturé issu de l'étape a) avec déshydratation concomitante conduisant à un nitrile insaturé.

10. Procédé selon l'une des revendications 8 et 9, **caractérisé en ce que** l'étape de nitrilation est réalisée en phase liquide ou en phase gaz au moyen d'ammoniac à une température généralement comprise entre 150°C et 350°C et avec un catalyseur.

11. Procédé selon la revendication 10, **caractérisé en ce que** ladite nitrilation est réalisée en phase liquide avec comme catalyseur un oxyde métallique qui est l'oxyde de zinc.

12. Procédé selon la revendication 10, **caractérisé en ce que** ladite nitrilation est réalisée en phase gaz avec ledit catalyseur étant supporté sur un lit fixe d'alumine dopée ou non.

13. Procédé selon l'une des revendications 8 à 12, **caractérisé en ce que** l'effluent de l'étape de nitrilation est soumis à l'étape c) de coupure oxydante, dont l'effluent (comprenant le composé) nitrile-acide est soumis à une hydrogénation d).

14. Procédé selon la revendication 13, **caractérisé en ce que** ladite hydrogénation est conduite à une température comprise entre 70 et 200°C, de préférence entre 70 et 150°C, plus préférentiellement entre 90 et 130°C, sous une pression de H₂ comprise entre 1 et 300 bars, de préférence entre 5 et 50 bars, en présence de catalyseurs d'hydrogénation, soit homogènes, soit hétérogènes.

15. Procédé selon l'une des revendications 1 à 14, **caractérisé en ce que** ledit acide ω-fonctionnalisé est un diacide ou un amino-acide et **en ce que** ladite charge d'origine naturelle contient des acides gras insaturés hydroxylés sous forme d'ester (d'alcool ou de polyol comme le glycérol ou glycérine) et en particulier d'huile hydroxylée d'acide gras correspondant (l'huile étant un ester de glycérol) avec lesdites étapes étant :
i) hydrogénation de l'ester hydroxylé, en particulier de l'huile correspondante
ii) déshydratation de l'ester hydroxylé hydrogéné, en particulier de l'huile correspondante
iii) hydrolyse de l'ester hydrogéné (et déshydraté), en particulier de l'huile correspondante pour obtenir l'acide gras insaturé correspondant et de l'alcool ou du polyol, en particulier de la glycérine
iv) séparation de l'alcool ou du polyol, en particulier de la glycérine (ou glycérol) et éventuellement séparation des isomères d'acide gras insaturés
v) en option (seulement dans le cas de nitrile), ammoniation de l'acide (gras) insaturé pour obtenir le nitrile insaturé correspondant
vi) coupure oxydante de l'acide gras ou nitrile insaturé (en fonction du cas)
vii) en option, séparation des produits de coupure et des acides légers formés
viii) en option (cas de nitrile), hydrogénation du nitrile-acide pour former l'amino-acide correspondant
ix) séparation et purification du diacide formé ou de l'amino-acide formé, en fonction du cas.

16. Procédé selon l'une des revendications 1 à 14, **caractérisé en ce que** ledit acide ω-fonctionnalisé est un amino-acide et **en ce que** ladite charge d'origine naturelle contient des acides gras insaturés hydroxylés sous forme d'ester (d'alcool ou de polyol comme le glycérol ou glycérine), en particulier d'une huile hydroxylée (ester de glycérol) et ledit procédé comprend l'enchaînement des étapes suivantes :
i) hydrogénation de l'ester hydroxylé, en particulier de l'huile correspondante
ii) hydrolyse de l'ester hydrogéné, en particulier de l'huile correspondante pour obtenir l'acide gras saturé correspondant et de l'alcool ou du polyol, en particulier de la glycérine
iii) séparation de l'alcool ou du polyol, en particulier de la glycérine avec éventuellement séparation des acides gras saturés des acides gras hydroxylés
iv) ammoniation de l'acide saturé hydroxylé, avec déshydratation simultanée, pour obtenir le nitrile insaturé correspondant
v) éventuellement (en option), séparation des isomères de nitriles insaturés
vi) coupure oxydante du nitrile insaturé
vii) en option, séparation des produits de coupure nitrile-acides et des acides légers formés
viii) hydrogénation du nitrile-acide, pour obtenir l'amino-acide correspondant
ix) séparation et purification de l'amino-acide obtenu.

17. Procédé selon l'une des revendications 1 à 14, **caractérisé en ce que** ledit acide ω-fonctionnalisé est un amino-acide via un nitrile acide, à partir d'une source d'huile végétale peu concentrée en acide hydroxylé, avec un enchaînement d'étapes suivantes :
i) alcoolyse (en particulier méthanolyse) de l'huile végétale, éventuellement concomitante à son extraction de la graine
ii) extraction d'une fraction riche en ester d'acide hydroxylé
iii) hydrogénation de l'ester hydroxylé
iv) en option, hydrolyse de l'ester hydrogéné pour obtenir l'acide gras correspondant
v) ammoniation de l'acide saturé hydroxylé avec déshydratation simultanée (y compris sur ester d'étape iii) ou acide d'étape iv)) pour obtenir le nitrile insaturé
vi) éventuellement, séparation des isomères de nitriles insaturés
vii) coupure oxydante du nitrile insaturé
viii) en option, séparation des produits de coupure nitrile-acides et des acides légers formés
ix) hydrogénation du nitrile-acide pour obtenir l'amino-acide
x) séparation et purification de l'amino-acide obtenu.

18. Procédé de préparation d'un nitrile gras insaturé comme matière première de coupure oxydante de l'étape c) telle que définie selon la revendication 1, **caractérisé en ce qu'**en plus des étapes a) et b) telles que définies selon la revendication 1, il comprend une étape de nitrilation soit séparée après l'étape b) soit en même temps que l'étape b) de déshydratation.

## Patentansprüche

1. Verfahren zur Synthese von ω-funktionalisierten Säuren, **dadurch gekennzeichnet, dass** die Säuren die Formel R-(CH₂)ₙ-COOH aufweisen, in der R für COOH oder NH₂CH₂ steht und n für eine ganze Zahl zwischen 9 und 12 steht, sie aus einem Einsatzstoff natürlicher Herkunft, der ungesättigte hydroxylierte Fettsäuren in der Säure-, Ester- oder Polyolester-Form mit mindestens 18 Kohlenstoffatomen pro Molekül enthält, erhalten werden und das Verfahren die folgenden Schritte umfasst:
a) Hydrierung der ungesättigten hydroxylierten Fettsäuren, was gesättigte hydroxylierte Fettsäuren ergibt,
b) Dehydratisierung der gesättigten hydroxylierten Fettsäuren, was einfach ungesättigte Fettsäuren ergibt,
c) oxidative Spaltung der Doppelbindung der einfach ungesättigten Fettsäuren, was eine α,ω-bifunktionelle Disäure- oder Nitrilsäure-Verbindung ergibt.

2. Verfahren nach Anspruch 1, **dadurch gekennzeichnet, dass** der Hydrierungsschritt a) bei einer Temperatur zwischen 70 und 180°C, vorzugsweise 70 und 150°C, weiter bevorzugt zwischen 90 und 130°C, unter einem H₂-Druck zwischen 1 und 300 bar, vorzugsweise zwischen 5 und 50 bar, in Gegenwart von entweder homogenen oder heterogenen Hydrierkatalysatoren durchgeführt wird.

3. Verfahren nach Anspruch 2, **dadurch gekennzeichnet, dass** es sich bei den Katalysatoren um Edelmetalle wie Pt, Pd oder Rh oder Übergangsmetalle wie Mo, W, Cr, Fe, Co oder Ni handelt, die allein oder als Mischung, eventuell in auf Aktivkohle, auf Aluminiumoxid und auf Siliciumdioxid geträgerter Form, verwendet werden.

4. Verfahren nach Anspruch 2 oder 3, **dadurch gekennzeichnet, dass** die Katalysatoren aus Raney-Nickel und/oder Palladium auf Aktivkohle ausgewählt werden.

5. Verfahren nach einem der Ansprüche 1 bis 4, **dadurch gekennzeichnet, dass** der Hydrierungsschritt a) unter solchen Arbeitsbedingungen durchgeführt wird, dass der Austragsstrom aus diesem Hydrierungsschritt eine Iodzahl < 5, vorzugsweise < 3 und weiter bevorzugt < 1 und eine Hydroxylzahl > 100 mg KOH/g aufweist.

6. Verfahren nach einem der Ansprüche 1 bis 5, **dadurch gekennzeichnet, dass** der Schritt b) der Dehydratisierung der gesättigten hydroxylierten Fettsäuren bei einer Temperatur zwischen 100 und 300°C und in Gegenwart eines Säurekatalysators, der vorzugsweise aus Schwefelsäure, Phosphorsäure, Sulfonsäuren, Alkylsulfonaten oder sauren Ionenaustauscherharzen ausgewählt wird, durchgeführt wird.

7. Verfahren nach einem der Ansprüche 1 bis 6, **dadurch gekennzeichnet, dass** der Schritt c) der oxidativen Spaltung unter Verwendung eines Oxidationsmittels, das aus KMnO₄, Wasserstoffperoxid oder oxidierendem Ozon ausgewählt wird, gegebenenfalls in Kombination mit einem Katalysator, wie Wolframsäure, insbesondere Ozon in Kombination mit Sauerstoff, durchgeführt wird.

8. Verfahren nach einem der Ansprüche 1 bis 7, **dadurch gekennzeichnet, dass** es einen zusätzlichen Zwischenschritt zwischen Schritt b) und Schritt c) der Nitrilierung der Säurefunktion der einfach ungesättigten Fettsäure, was ein ungesättigtes Nitril ergibt, umfasst.

9. Verfahren nach einem der Ansprüche 1 bis 7, **dadurch gekennzeichnet, dass** es einen Schritt der Nitrilierung der Säurefunktion der gesättigten hydroxylierten Fettsäure aus Schritt a) mit gleichzeitiger Dehydratisierung, was ein ungesättigtes Nitril ergibt, umfasst.

10. Verfahren nach einem der Ansprüche 8 und 9, **dadurch gekennzeichnet, dass** der Nitrilierungsschritt in der Flüssigphase oder in der Gasphase mit Hilfe von Ammoniak bei einer Temperatur, die im Allgemeinen zwischen 150°C und 350°C liegt, und mit einem Katalysator durchgeführt wird.

11. Verfahren nach Anspruch 10, **dadurch gekennzeichnet, dass** die Nitrilierung in der Flüssigphase mit einem Metalloxid, bei dem es sich um Zinkoxid, handelt, als Katalysator durchgeführt wird.

12. Verfahren nach Anspruch 10, **dadurch gekennzeichnet, dass** die Nitrilierung in der Gasphase durchgeführt wird, wobei der Katalysator auf einem Festbett aus gegebenenfalls dotiertem Aluminiumoxid geträgert ist.

13. Verfahren nach einem der Ansprüche 8 bis 12, **dadurch gekennzeichnet, dass** der Austragsstrom aus dem Nitrilierungsschritt dem Schritt c) der oxidativen Spaltung unterworfen wird, dessen Austragsstrom (der die Nitrilsäure-Verbindung umfasst) einer Hydrierung d) unterworfen wird.

14. Verfahren nach Anspruch 13, **dadurch gekennzeichnet, dass** die Hydrierung bei einer Temperatur zwischen 70 und 200°C, vorzugsweise 70 und 150°C, weiter bevorzugt zwischen 90 und 130°C, unter einem H₂-Druck zwischen 1 und 300 bar, vorzugsweise zwischen 5 und 50 bar, in Gegenwart von entweder homogenen oder heterogenen Hydrierkatalysatoren durchgeführt wird.

15. Verfahren nach einem der Ansprüche 1 bis 14, **dadurch gekennzeichnet, dass** es sich bei der ω-funktionalisierten Säure um eine Disäure oder eine Aminosäure handelt und der Einsatzstoff natürlicher Herkunft hydroxylierte ungesättigte Fettsäuren in Esterform (Alkohol- oder Polyolester wie Glycerol- bzw. Glycerinester) und insbesondere in Form von hydroxyliertem Öl der entsprechenden Fettsäure (wobei es sich bei dem Öl um einen Glycerolester handelt) enthält, wobei es sich bei den Schritten um Folgende handelt:
i) Hydrierung des hydroxylierten Esters, insbesondere des entsprechenden Öls,
ii) Dehydratisierung des hydrierten hydroxylierten Esters, insbesondere des entsprechenden Öls,
iii) Hydrolyse des hydrierten (und dehydratisierten) Esters, insbesondere des entsprechenden Öls, zum Erhalt der entsprechenden ungesättigten Fettsäure und des Alkohols bzw. Polyols, insbesondere von Glycerin,
iv) Abtrennung des Alkohols bzw. Polyols, insbesondere des Glycerins (bzw. Glycerols), und eventuell Trennung der ungesättigten Fettsäureisomere,
v) gegebenenfalls (nur im Fall von Nitril) Ammonisierung der ungesättigten (Fett)Säure zum Erhalt des entsprechenden ungesättigten Nitrils,
vi) oxidative Spaltung der ungesättigten Fettsäure bzw. des ungesättigten Nitrils,
vii) gegebenenfalls Trennung der gebildeten Spaltungsprodukte und leichten Säuren,
viii) gegebenenfalls (Fall von Nitril) Hydrierung der Nitrilsäure zur Bildung der entsprechenden Aminosäure,
ix) Abtrennung und Reinigung der gebildeten Disäure bzw. der gebildeten Aminosäure.

16. Verfahren nach einem der Ansprüche 1 bis 14, **dadurch gekennzeichnet, dass** es sich bei der ω-funktionalisierten Säure um eine Aminosäure handelt und der Einsatzstoff natürlicher Herkunft hydroxylierte ungesättigte Fettsäuren in Esterform (Alkohol- oder Polyolester wie Glycerol- bzw. Glycerinester) und insbesondere in Form von hydroxyliertem Öl (Glycerolester) enthält und das Verfahren die Abfolge der folgenden Schritte umfasst:
i) Hydrierung des hydroxylierten Esters, insbesondere des entsprechenden Öls,
ii) Hydrolyse des hydrierten Esters, insbesondere des entsprechenden Öls, zum Erhalt der entsprechenden gesättigten Fettsäure und des Alkohols bzw. Polyols, insbesondere von Glycerin,
iii) Abtrennung des Alkohols bzw. Polyols, insbesondere des Glycerins, mit eventueller Trennung der gesättigten Fettsäuren von den hydroxylierten Fettsäuren,
iv) Ammonisierung der hydroxylierten gesättigten Säure mit gleichzeitiger Dehydratisierung zum Erhalt des entsprechenden ungesättigten Nitrils,
v) eventuell (gegebenenfalls) Trennung der ungesättigten Nitrilisomere,
vi) oxidative Spaltung des ungesättigten Nitrils,
vii) gegebenenfalls Trennung der gebildeten Nitrilsäure-Spaltungsprodukte und leichten Säuren,
viii) Hydrierung der Nitrilsäure zum Erhalt der entsprechenden Aminosäure,
ix) Abtrennung und Reinigung der erhaltenen Aminosäure.

17. Verfahren nach einem der Ansprüche 1 bis 14, **dadurch gekennzeichnet, dass** es sich bei der ω-funktionalisierten Säure um eine Aminosäure über eine Nitrilsäure ausgehend von einer Pflanzenölquelle mit einer geringen Konzentration an hydroxylierter Säure handelt, mit einer Abfolge der folgenden Schritte:
i) Alkoholyse (insbesondere Methanolyse) des Pflanzenöl, eventuell gleichzeitig mit dessen Extraktion aus dem Samen,
ii) Extraktion einer an hydroxyliertem säureester reichen Fraktion,
iii) Hydrierung des hydroxylierten Esters,
iv) gegebenenfalls Hydrolyse des hydrierten Esters zum Erhalt der entsprechenden Fettsäure,
v) Ammonisierung der hydroxylierten gesättigten Säure mit gleichzeitiger Dehydratisierung (einschließlich des Esters aus Schritt iii) bzw. der Säure aus Schritt iv)) zum Erhalt des ungesättigten Nitrils,
vi) eventuell Trennung der ungesättigten Nitrilisomere,
vii) oxidative Spaltung des ungesättigten Nitrils,
viii) gegebenenfalls Trennung der gebildeten Nitrilsäure-Spaltungsprodukte und leichten Säuren,
ix) Hydrierung der Nitrilsäure zum Erhalt der entsprechenden Aminosäure,
ix) Abtrennung und Reinigung der erhaltenen Aminosäure.

18. Verfahren zur Herstellung eines ungesättigten Fettnitrils als Ausgangsstoff für die oxidative Spaltung von Schritt c) gemäß Anspruch 1, **dadurch gekennzeichnet, dass** es neben den Schritten a) und b) gemäß Anspruch 1 einen Nitrilierungsschritt umfasst, der entweder separat nach Schritt b) oder gleichzeitig mit dem Dehydratisierungsschritt b) durchgeführt wird.

## Claims

1. Process for the synthesis of ω-functionalized acids, **characterized in that** said acids are of formula R-(CH₂)ₙ-COOH, in which R represents COOH or NH₂CH₂ and n represents an integer between 9 and 12, they are obtained from a feedstock of natural origin comprising unsaturated hydroxylated fatty acids in the acid, ester or polyol ester form comprising at least 18 carbon atoms per molecule and **in that** said process comprises the following stages:
a) hydrogenation of the unsaturated hydroxylated fatty acids, resulting in saturated hydroxylated fatty acids,
b) dehydration of the saturated hydroxylated fatty acids, resulting in monounsaturated fatty acids,
c) oxidative cleavage at the double bond of the monounsaturated fatty acids, resulting in an α,ω-bifunctional compound from diacid or nitrile-acid.

2. Process according to Claim 1, **characterized in that** the hydrogenation stage a) is carried out at a temperature of between 70 and 180°C, preferably 70 and 150°C, more preferably between 90 and 130°C, under an H₂ pressure of between 1 and 300 bar, preferably between 5 and 50 bar, in the presence of either homogeneous or heterogeneous hydrogenation catalysts.

3. Process according to Claim 2, **characterized in that** said catalysts are noble metals, such as Pt, Pd or Rh, or transition metals, such as Mo, W, Cr, Fe, Co or Ni, used alone or as a mixture, optionally in the form supported on active charcoal, on alumina and on silica.

4. Process according to Claim 2 or 3, **characterized in that** said catalysts are chosen from Raney nickel and/or palladium-on-active charcoal.

5. Process according to one of Claims 1 to 4, **characterized in that** the hydrogenation stage a) is carried out under operational conditions such that the effluent resulting from this hydrogenation stage exhibits an iodine number < 5, preferably < 3 and more preferably < 1 and a hydroxyl number > 100 mg KOH/g.

6. Process according to one of Claims 1 to 5, **characterized in that** the stage b) of dehydration of the saturated hydroxylated fatty acids is carried out at a temperature of between 100 and 300°C and in the presence of an acid catalyst, preferably chosen from: sulfuric acid, phosphoric acid, sulfonic acids, alkyl sulfonates or ionexchange acid resins.

7. Process according to one of Claims 1 to 6, **characterized in that** the oxidative cleavage stage c) is carried out using an oxidizing agent chosen from KMnO₄, hydrogen peroxide or oxidizing ozone, optionally in combination with a catalyst, such as tungstic acid, in particular ozone in combination with oxygen.

8. Process according to one of Claims 1 to 7, **characterized in that** it comprises an additional intermediate stage, between stage b) and stage c), of nitrilation of the acid functional group of the monounsaturated fatty acid, resulting in an unsaturated nitrile.

9. Process according to one of Claims 1 to 7, **characterized in that** it comprises a stage of nitrilation of the acid functional group of the saturated hydroxylated fatty acid resulting from stage a) with concomitant dehydration, resulting in an unsaturated nitrile.

10. Process according to either of Claims 8 and 9, **characterized in that** the nitrilation stage is carried out in the liquid phase or in the gas phase using ammonia at a temperature generally of between 150°C and 350°C and with a catalyst.

11. Process according to Claim 10, **characterized in that** said nitrilation is carried out in the liquid phase with, as catalyst, a metal oxide which is zinc oxide.

12. Process according to Claim 10, **characterized in that** said nitrilation is carried out in the gas phase with said catalyst being supported on a fixed bed of doped or non-doped alumina.

13. Process according to one of Claims 8 to 12, **characterized in that** the effluent from the nitrilation stage is subjected to the oxidative cleavage stage c), the effluent (comprising the nitrile-acid compound) of which is subjected to a hydrogenation d).

14. Process according to Claim 13, **characterized in that** said hydrogenation is carried out at a temperature of between 70 and 200°C, preferably between 70 and 150°C and more preferably between 90 and 130°C, under an H₂ pressure of between 1 and 300 bar, preferably between 5 and 50 bar, in the presence of either homogeneous or heterogeneous hydrogenation catalysts.

15. Process according to one of Claims 1 to 14, **characterized in that** said ω-functionalized acid is a diacid or an amino acid and **in that** said feedstock of natural origin comprises unsaturated hydroxylated fatty acids in the ester form (alcohol or polyol ester, such as glycerol ester) and in particular in the form of hydroxylated oil of corresponding fatty acid (the oil being a glycerol ester), with said stages being:
i) hydrogenation of the hydroxylated ester, in particular the corresponding oil
ii) dehydration of the hydrogenated hydroxylated ester, in particular of the corresponding oil
iii) hydrolysis of the hydrogenated (and dehydrated) ester, in particular of the corresponding oil, in order to obtain the corresponding unsaturated fatty acid and alcohol or polyol, in particular glycerol
iv) separation of the alcohol or polyol, in particular glycerol, and optionally separation of the unsaturated fatty acid isomers
v) optionally (only in the case of nitrile), ammoniation of the unsaturated (fatty) acid in order to obtain the corresponding unsaturated nitrile
vi) oxidative cleavage of the unsaturated nitrile or fatty acid (as the case may be)
vii) optionally, separation of the cleavage products and light acids formed
viii) optionally (case of nitrile), hydrogenation of the nitrile-acid in order to form the corresponding amino acid
ix) separation and purification of the diacid formed or of the amino acid formed, as the case may be.

16. Process according to one of Claims 1 to 14, **characterized in that** said ω-functionalized acid is an amino acid and **in that** said feedstock of natural origin comprises unsaturated hydroxylated fatty acids in the ester (alcohol or polyol ester, such as glycerol ester) form, in particular in the form of a hydroxylated oil (glycerol ester), and said process comprises the sequence of the following stages:
i) hydrogenation of the hydroxylated ester, in particular of the corresponding oil
ii) hydrolysis of the hydrogenated ester, in particular of the corresponding oil, in order to obtain the corresponding saturated fatty acid and alcohol or polyol, in particular glycerol
iii) separation of the alcohol or polyol, in particular glycerol, with optional separation of the saturated fatty acids from the hydroxylated fatty acids
iv) ammoniation of the saturated hydroxylated acid, with simultaneous dehydration, in order to obtain the corresponding unsaturated nitrile
v) optionally, separation of the unsaturated nitrile isomers
vi) oxidative cleavage of the unsaturated nitrile
vii) optionally, separation of the nitrile-acid cleavage products and of the light acids formed
viii) hydrogenation of the nitrile-acid, in order to obtain the corresponding amino acid
ix) separation and purification of the amino acid obtained.

17. Process according to one of Claims 1 to 14, **characterized in that** said ω-functionalized acid is an amino acid via a nitrile-acid, starting from a vegetable oil source which has a low concentration of hydroxylated acid, with a sequence of following stages:
i) alcoholysis (in particular methanolysis) of the vegetable oil, optionally concomitant with its extraction from the seed
ii) extraction of a fraction rich in hydroxylated acid ester
iii) hydrogenation of the hydroxylated ester
iv) optionally, hydrolysis of the hydrogenated ester in order to obtain the corresponding fatty acid
v) ammoniation of the saturated hydroxylated acid with simultaneous dehydration (including on ester of stage iii) or on acid of stage iv)), in order to obtain the unsaturated nitrile
vi) optionally, separation of the unsaturated nitrile isomers
vii) oxidative cleavage of the unsaturated nitrile
viii) optionally, separation of the nitrile-acid cleavage products and of the light acids formed
ix) hydrogenation of the nitrile-acid in order to obtain the amino acid
x) separation and purification of the amino acid obtained.

18. Process for the preparation of an unsaturated fatty nitrile as starting material for the oxidative cleavage of stage c) as defined according to Claim 1, **characterized in that**, in addition to stages a) and b) as defined according to Claim 1, it comprises a nitrilation stage either separated after the dehydration stage b) or at the same time as the dehydration stage b).
